# EUROPEAN PATENT APPLICATION

(11) **EP 4 275 639 A1**
(43) Date of publication of application: **15.11.2023**
(21) Application number: 22172473.5
(22) Date of filing: 10.05.2022
(51) Int. Cl.: A61B 34/10, A61B 34/20, A61B 17/34, A61B 90/00

(54) **SYSTEM AND METHOD FOR ASSISTANCE IN A SURGICAL PROCEDURE**

(71) Applicant: Koninklijke Philips N.V., 5656 AG Eindhoven (NL)
(72) Inventor: SCHMIDT-RICHBERG, Alexander, Eindhoven (NL); PREVRHAL, Sven Peter, Eindhoven (NL); WIEMKER, Rafael, Eindhoven (NL); KLINDER, Tobias, Eindhoven (NL); STROOSMA, Otto, Eindhoven (NL); GERAATS, Jacobus Sigbertus Marie, Eindhoven (NL)
(74) Representative: Philips Intellectual Property & Standards

(57) **Abstract**

The invention relates to a system and to a method for assistance in an image guided surgical procedure, in particular in image guided percutaneous needle interventions such as for kidney stone removal. With the system and the method, it may be possible to provide an automatic recommendation of control scans at critical phases during the intervention. To this end, in an initial image, a structure at risk is identified and an intervention path to a target location is provided. With an evaluation unit, it can be evaluated whether a spatial relation between a) a tracked position of a medical instrument and b) the location of the structure at risk and/or the intervention path meets a predefined criterion. If the predefined criterion is found to be met, an acquisition of a control scan may be recommended automatically.

## Description

### FIELD OF THE INVENTION

The invention relates to a system for assistance in a surgical procedure, to a method for assistance in a surgical procedure, to a computer program, and to a non-transitory computer readable data medium.

### BACKGROUND OF THE INVENTION

In minimal surgery, it is known to plan a priori an incision point and a trajectory to an anatomical region in a patient's body. To this end, generally prior recorded fluoroscopic or tomographic images showing the anatomical region of the patient are used. During surgery, typically, continuous low-dose x-ray imaging or x-ray imaging at selected times is performed to verify the position of a medical instrument with control images while guiding the medical instrument along the planned path to the anatomical region of the patient. As a result, the patient and sometimes even a clinician conducting the surgery are exposed to some extend to x-ray radiation during surgery.

To reduce the x-ray exposure, it was suggested to use a robot to perform the surgical procedure based on an anatomical roadmap. An according setup making use of an interventional robot is disclosed in WO 2019/092225 A1. Therein, an intervention system is disclosed that shall facilitate a reduction in radiation exposure in an interventional setting by providing an interventional controller for a robotic navigation of an interventional instrument based on a single static anatomical roadmap that is automatically updated by the interventional controller when the robot reaches a critical navigation position within the anatomical roadmap and/or when a physiological status of the patient is critical.

Yet, it is still desirable to further improve patient safety during a surgical procedure with, at the same time, a reduced x-ray exposure of the patient and, if present, of clinicians during surgery.

### SUMMARY OF THE INVENTION

The present invention is based on the objective of providing a system, a method, a computer program and a non-transitory computer readable data medium for assistance in conducting a surgical procedure with an increased reliability. Preferably, with the system, the method, the computer program and the non-transitory computer readable data medium it is furthermore possible to conduct a surgical procedure with a comparatively reduced x-ray exposure of a patient.

With regard to the system, a system for assistance in a surgical procedure is proposed that comprises an image providing unit, an intervention path providing unit, a structure at risk providing unit, a position providing unit, an evaluation unit, and an output unit. The image providing unit is configured for providing an initial image of a target location inside a patient's body. The intervention path providing unit is configured for providing in the initial image an intervention path along which a medical instrument shall be guided to the target location. The structure at risk providing unit is configured for providing in the initial image a location of a structure at risk. The position providing unit is configured for providing a tracked position of the medical instrument. The evaluation unit is configured for evaluating whether a spatial relation between a) the tracked position and b) the location of the structure at risk and/or the intervention path meets a predefined criterion. The output unit is configured for providing an output signal indicative of whether the spatial relation meets the predefined criterion.

The invention includes the recognition that for increasing the reliability of a surgical procedure it is beneficial to plan the surgical procedure a priori using an initial image of a target location inside a patient's body. Based on the initial image of the target location, the intervention path along which a medical instrument shall be guided to the target location can be planned. It is furthermore of advantage if the position of the medical instrument inside the patient can be tracked and to evaluate the tracked position of the medical instrument in relation to the planned intervention path.

The invention now includes the further recognition that on its way to the target location inside the patient's body, the medical instrument typically will traverse areas inside the patient that are less critical such that a slight deviation from the intervention path will most probably not cause any severe damage to the patient. However, there may also be one or more structures at risk in the initial image that may lie critically close to the intervention path. A structure at risk may be a structure that should not be damaged, e.g., due to puncturing, by the medical instrument to avoid more severe damage to the patient. With the system, it is possible to use a tracked position of the medical instrument for evaluating a spatial relation between the tracked medical instrument and location of the structure at risk and/or the intervention path. In particular, with the system's evaluation it can be evaluated whether the spatial relation meets a predefined criterion. As a result of the evaluation of the medical instrument's tracked position and the location of the structure at risk and/or the intervention path, it is possible to decide whether the predefined criterion is met. Based on whether the predefined criterion is met it is possible to perform some sort of correction of the surgical procedure or to proceed with the surgical procedure with increased confidence. The evaluation of the spatial relation between the tracked position and the location of the structure at risk and/or the intervention path with respect to a predefined criterion thus improves the reliability of the surgical procedure and reduces the risk of damaging a structure at risk of a patient. The system thereby assists in safely guiding a medical instrument to a target location inside a patient without damaging nearby structures at risk. Thereby, the system allows increasing a success rate of a surgical procedure and helps to improve the health condition of patients.

For providing the initial image, the image providing unit may be configured for receiving the initial image from another external device, e.g., an imaging device. It is also possible that the image providing unit is configured for generating the initial image, e.g., the image providing unit itself can be an imaging device.

The initial image may be a fluoroscopic image, a tomographic image, or a sonographic image. Accordingly, the initial image may be generated by a fluoroscopic imaging device such as a C-arm that has an x-ay source and an x-ray detector. The initial image may also be generated by an x-ray computed tomography (CT) device such as a cone beam computed tomography (CBCT) device. The initial image may also be generated by a sonographic device. Preferably, the initial image is captured prior to a surgical procedure, preferably, as a planning image. The initial image may also be captured directly at the beginning of a surgical procedure. It may also be possible that the initial image is recorded during a surgical procedure, e.g., to adapt a surgical plan.

The tracked position of the medical instrument as provided by the system's position providing unit can be generated by tracking system and provided to the position providing unit. In particular, the position providing unit may be configured to receive the tracked position from a tracking system. A tracking system can be an optical tracking system. An optical tracking system typically uses infrared light to determine the position of a medical instrument equipped with active markers such as light emitting diodes (LEDs) or passive maskers such as spheres or disks coated with a highly reflective material that are attached to the medical device and detected using a camera. A tracking system can be an electromagnetic tracking system. An electromagnetic tracking system generally comprises a filed generator for generating an electromagnetic field and electromagnetic sensors attached to the medical instrument for tracking. For using the tracked position of the medical instrument as tracked with the tracking system, the tracked position defined in the coordinate system of the tracking system has to be transformed into the coordinate system of the initial image. This includes patient registration and calibration of the medical instrument. In more general terms, tracking of a position of marker disposed on proximal end of a medical instrument using a non-imaging tracking system, e.g. based on an optical marker or an electromagnetic marker may be accomplished by registering the tracked position to a reference X-ray image of a patient. Subsequently, a position of a distal end of the interventional device with respect to a critical structure in the subject based on the tracked position of the marker may be estimated. It is also possible to track the position by means of a fixed physical guidance, e.g., a jig, between the imaging device and the medical instrument.

It is also possible to track the position of the medical instrument, and thus to provide the tracked position using a mobile application on a standard mobile consumer device, e.g., a smart phone or a tablet computer, which relies on a database of one or more digital computer-aided design (CAD) geometries of the medical instrument and a casing of an employed medical imaging device. With the mobile application, a geometric inter-relation, e.g., the 3D positions of the imaging device and the medical instrument, may be established by matching point clouds yielded, e.g., by light detection and ranging (LIDAR) or a similar depth-ranging camera system. Alternatively to using a LIDAR or the like, the 3D positions of imaging device and the medical instrument may be extracted from a 2D image feed, i.e., of a webcam or the like. This approach is based on the recognition that if the shape of the imaging device, e.g., of a CBCT, and the shape of the medical instrument can be recognized in an image feed of a detector, then the spatial relationship between the imaging device and the medical instrument may be extracted. Since the position of the imaging device may be known, for instance, a CBCT gantry position can be extracted from a CBCT control computer, and the spatial relationship of the gantry to the captured images may also be known, it may be possible to also determine the position and orientation of the medical instrument.

The medical instrument the tracked position of which is provided with the position providing unit may be a needle or a catheter or a guidewire or some sort of other access channel, e.g., for an endoscope or the like. In particular, the medical instrument may be an instrument used for insertion into a patient as part of a surgical procedure. The medical instrument can be a handheld device that is to be guided by a human or it can be a medical instrument that is to be guided by a robot.

A structure at risk may be a human organ such as a kidney, a heart, lung or the like or a vessel like a blood vessel, e.g., a vein, or the like.

In general, the components of the system, i.e., in particular, the image providing unit, the intervention path providing unit, the structure at risk providing unit, the evaluation unit, and the output unit can be configured by hardware and software that is adapted for carrying out the respective functionality. For example, the aforementioned components of the system can be implemented as part of a personal computer or other computing device or the like.

Preferably, the system further comprises a control unit configured for controlling the image providing unit to provide a control image of the target location if the output signal indicates that the evaluated spatial relation meets the predefined criterion.

Thereby, based on the evaluated spatial relation, the control unit may provide an automatic recommendation of when to perform a control scan. The control unit may trigger the image providing unit to generate a control image, i.e., to capture the control image by itself or by requesting an external imaging device to provide the control image. Hence, the control image is only provided in certain, e.g., critical, situations that may necessitate to evaluate the location of the tracked medical instrument with respect to certain body parts, e.g., structures at risk. Thereby, the number of control scans can be limited to a minimum number that is required to ensure a safe guidance of the medical instrument to the target location. Advantageously, by means of the control unit it can thus be avoided to provide a control scan in a situation where a control scan actually would not be needed. For example, a recommendation to acquire an updated X-ray control image as provided by the control unit may be based on a proximity of the estimated tracked position of the distal end of the medical instrument with respect to a location of a structure at risk.

Advantageously, using a control unit allows replacing continuous x-ray fluoroscopy imaging with a dose-saving regime of intermittent single-view x-ray exposures at a few time points, i.e., control exposures, selected by the control unit based on the evaluation of the spatial relation between tracked position and the intervention path and/or the location of structures at risk.

Preferably, the control unit may control the image providing unit based on the output signal provided by the output unit and being indicative of whether the spatial relation meets the predefined criterion. Based on whether the spatial relation meets the predefined criterion, the control unit may or may not request the image providing unit to provide a control image.

The control image may be used as an new initial image by the system, i.e., the intervention path providing unit may provide in the new initial image a new intervention path along which the medical instrument shall be guided to the target location, and the structure at risk providing unit may provide in the new initial image a new location of a structure at risk. The new initial image may then be used by the evaluation unit for evaluation purposes. It is possible, that the initial image is, e.g., a fluoroscopic image, and the control image that may be used as a new initial image, is a tomographic or sonographic image, or vice versa.

In particular, the control image may show changes to the scene captured in the initial image, e.g., since the patient may have moved or the interaction between medical instrument and the patient's anatomy may have caused a shift in the anatomy, e.g., the medical instrument may have pushed an organ or another structure at risk to the side.

Alternatively or additionally, the control unit may be configured for defining a control area at which the predefined criterion is met and for controlling the image providing unit to provide a control image when the medical instrument reaches the control area.

A control area, preferably, is defined in the initial image and indicates a position of the medical instrument at which the predefined criterion is met. A control area thus indicates a location inside the patient at which the spatial relation meets the predefined criterion such that a control image should be captured. The control area thus indicate a position of the medical instrument at which a control image has been or will be captured. It is preferred that a control area is defined prior to surgery in the initial image. However, it may also be beneficial if a control area is defined during surgery, e.g., based on a control image.

For example, a control area may be defined relative to a structure at risk. In particular, a control area may be positioned on the intervention path such that if the tracked medical instrument is expected to reach the control area based on the tracked position, the control unit may trigger the image providing unit to provide a control image. The control area may have the geometry of a point, a sphere or a 3D volume. Its dimension can be selected according to its position relative to, e.g., a structure at risk. For example, if the control area is located close to a structure at risk, it may be defined to have a large dimension compared to a situation in which a structure at risk is further away from the intervention path. The control unit may then trigger the image providing unit to provide a control image comparatively earlier, i.e., further away from the structure at risk if a critical spatial relation between medical instrument and the structure at risk is expected.

The system may further comprise a visualization unit configured for visualizing the tracked position and the control area in the initial image. The control area, preferably, indicates a position of the medical instrument at which the control image has been or will be provided. The visualization unit may further be configured for visualizing the intervention path and wherein the control area lies on the intervention path. For example, the visualization unit may be or may comprise a graphical user interface. The visualization unit may be further configured for visualizing the provided structure at risk. The visualization unit may thus visualize the planned intervention path as provided, e.g., by the intervention path providing unit together with the structure at risk. During surgery, the visualization unit may also visualize the tracked position of the medical instrument, e.g., relative to the intervention path.

Preferably, the structure at risk providing unit is configured for defining a safety distance with respect to the structure at risk. Preferably, the evaluation unit is configured for evaluating that the spatial relation between a) the tracked position and b) the location of the structure at risk meets the predefined criterion if the tracked position of the medical instrument is closer to the structure at risk than the defined safety distance.

It is a particular purpose of the safety distance to define a buffer zone with respect to the structure at risk such that a control image may be provided already when the medical instrument reaches or is expected to reach the safety distance. In this case, the control image is provided before actually reaching the structure at risk. Thereby, the risk of puncturing a structure at risk can be reduced. For example, a control area may be located at the safety distance such that the control unit may control the image providing unit to provide a control image when based on the provided tracked position, the medical instrument is expected to have reached the control area.

The safety distance may translate to a safety margin around the structure at risk or in 3D to a safety envelope enclosing the structure at risk.

For defining the safety margin, an expected relative movement caused by positioning the patient prior to an intervention and/or an expected movement caused by breathing of the patient during the intervention can be considered. The structure at risk providing unit may be configured for defining a safety margin around the structure at risk and wherein the intervention path providing unit may be configured for providing the intervention path such that the intervention path does not intersect the structure at risk and the defined safety margin.

Alternatively or additionally, the structure at risk providing unit may be configured for defining a safety distance with respect to the structure at risk and for determining an intersection between the provided intervention path and the defined safety distance. Preferably, the evaluation unit is configured for evaluating that the spatial relation between a) the tracked position and b) the location of the structure at risk and the intervention path meets the predefined criterion if the tracked position is closer to the intersection than a predefined threshold intersection distance.

In this case, the intervention path intersects the safety distance such that the intervention part is provided to be closer to a structure at risk than the extension of the safety distance. With respect to the intersection between intervention path and safety distance, an intersection distance may be defined. If the tracked position is closer to the intersection than a predefined threshold intersection distance, the medical instrument is expected to move relatively close, i.e., closer than the extension of the safety distance, to the structure at risk. Yet, since the evaluation unit is configured to use as the predefined criterion if the tracked position is closer to the intersection than a predefined threshold intersection distance, an output signal may be provided that may be used by the control unit for triggering the image providing unit to provide a control image. It is thus possible to evaluate the actual spatial relation between the medical instrument and a structure at risk to safely guide the medical instrument without puncturing the structure at risk. In particular, the intersection is an intersection point with a predefined diameter. The intersection itself may be used as a predefined or planned control area.

Preferably, the evaluation unit is configured for evaluating that the spatial relation between a) the tracked position and b) the intervention path meets the predefined criterion if a deviation of the tracked position from the intervention path exceeds a threshold deviation value indicative of an allowed deviation of the tracked position from the intervention path. The threshold deviation value thus defines how far the tracked position is allowed to deviate, before it is considered necessary to interrupt the surgical procedure, e.g., for providing a control image.

Alternatively or additionally, the evaluation unit may be configured for evaluating if a reliability value associated with the tracked position is below a threshold reliability value for the tracked position and the output unit may be further configured for providing a warning signal if the reliability value is below the threshold reliability value.

The threshold reliability value thus defines how well a tracked position is expected to represent the real position of the medical instrument relative to a patient. It may be that a certain degree of deviation of the tracked position from the real position is allowable, however, if the deviation of the tracked position from the real position becomes too large, the tracked position cannot be used anymore with sufficient reliability during a surgical procedure. The transition from an allowable deviation to an unallowable deviation is defined by the threshold reliability value and may be chosen according to, e.g., the type of surgical procedure to be carried out or, e.g., according to a specific anatomy of a patient.

For evaluating a reliability of the tracked position, the tracked position as provided by the position providing unit may be additionally provided with an associated reliability value that can be compared to the threshold reliability value. The reliability value for a tracked position may be generated by the position providing unit or by a tracking system. For example, for generating a reliability value, formerly tracked positions may be compared to the current tracked position to determine whether the current tracked position lies on the same path as the formerly tracked positions. If the tracked position was determined by an electromagnetic tracking system, also detected distortions in the generated electromagnetic field may be considered for determining a reliability value for a tracked position. Based on a provided reliability value it is thus possible to detect whether a tracked position is reliable. If the reliability value indicates that the tracked position is comparatively reliable, i.e. lying above the threshold reliability value, it can be assumed that the medical instrument is on the intervention path. A sufficiently reliable tracked position also implies that planned control areas can be used, e.g., for avoiding puncturing of structures at risk. Otherwise, if the comparison of a reliability value of a tracked position to the threshold reliability value yields that there is a high uncertainty about the tracked position, an additional control image may be provided that is not triggered by a prior set control area. Based on the control image, the tracking may be triggered to re-calibrate the medical instrument. The threshold for triggering a re-calibration may depend on a distance to a structure at risk such as an organ. Accordingly, if the reliability value is below the threshold reliability value, a control image may be captured to verify the position of the medical instrument.

In particular, the control unit may further be configured for controlling the image providing unit to provide a control image based on the provided warning signal. A warning signal may also trigger a visual signal or an audio signal indicative of the reliability value being below the threshold reliability value. The warning signal may be used to warn a clinician that the reliability of the tracked position is low.

Alternatively or additionally, the evaluation unit may be configured for evaluating that the spatial relation between a) the tracked position and b) the intervention path meets the predefined criterion if the tracked position is closer to the target location than a threshold target distance to the target location.

The threshold target distance can also be zero such that the spatial relation between a) the tracked position and b) the intervention path meets the predefined criterion if the tracked position is at the target location. Yet, it is preferred that the threshold target distance is larger than zero. If the tracked position is closer to the target location than a threshold target distance to the target location, a control image may be captured before actually reaching the target location. Preferably, the intervention path ends at the target location, i.e., the intervention path may connect an incision point with the target location. Therefore, if the medical instrument reaches the end of the intervention path, at the same time, it arrives at the target location. The threshold target distance preferably is defined with respect to the intervention path and, for example, comprise an end section of the intervention path. Thus, prior to reaching the end of the intervention path, the tracked position may be closer to the target location than a threshold target distance. Therefore, before actually reaching the target location, the predefined criterion is met and a control image may be provided. For example, the target location may be within a structure of risk, e.g., an organ, and the threshold target distance may be defined such that it represents the distance between the target location and the outer surface of the structure at risk. In this case, the predefined criterion is met when the medical instrument enters the structure at risk prior to reaching the target location inside. In one example, the target location may be a kidney stone to be removed from a kidney that is a structure of risk via minimal invasive surgery. The threshold target distance may be chosen to represent the distance from the kidney stone to the outer surface of the kidney such that the predefined criterion is met when the medical instrument enters the kidney prior to reaching the kidney stone to be removed.

Preferably, the structure at risk providing unit comprises a neural network that is trained for receiving the initial image as input and for providing as output a location of the structure at risk in the initial image. The trained neural network can be a multi-scale neural network or a recurrent neural network (RNN) such as, but not limited to, a gated recurrent unit (GRU) recurrent neural network or a long short-term memory (LSTM) recurrent neural network. Alternatively, the neural network may be a convolutional neural network (CNN). The training data used for training the neural network may represent images of structures at risk that have been previously identified. Thereby the neural network can be adapted for identifying structures at risk in a provided initial image.

According to the invention, furthermore, a method for assistance in a surgical procedure is proposed, said method comprising the steps of:
- providing an initial image of a target location inside a patient's body,
- providing in the initial image an intervention path for guiding a medical instrument to the target location,
- providing in the initial image a location of a structure at risk,
- providing a tracked position of the medical instrument,
- determining a spatial relation between the tracked position and the planned intervention path with the determination unit,
- evaluating whether a spatial relation between a) the tracked position and b) the location of the structure at risk and/or the intervention path meets a predefined criterion, and
- providing an output signal indicative of whether the spatial relation meets the predefined criterion.

The order of the steps:
- providing in the initial image an intervention path for guiding a medical instrument to the target location, and
- providing in the initial image a location of a structure at risk,
   may be reversed in different variants of the method. The method according to the invention can be conducted using the above-described system for assistance in a surgical procedure.

The method may further comprise the step of
- providing a control image of the target location if the output signal indicates that the evaluated spatial relation meets the predefined criterion.

According to the invention, furthermore, a computer program is proposed including instructions for executing the steps of the above-defined method, when run on a computer. The computer program may be stored on a non-transitory computer readable data medium.

In summary, the invention relates to using a tracked position of a medical instrument to estimate a position of a distal end of the medical instrument in relation to a structure at risk, and recommending - if the tracked position of the distal end is too close to a structure at risk - the acquisition of an updated control image of a target location.

In particular, the above described system and the method for assistance in a surgical procedure may be employed in conjunction with a plurality of surgical procedures where a 3D planning imaging exam is conducted before surgery or where it could be helpful to do so, and in particular to minimally invasive surgery where surgical access, i.e. the incision point and the trajectory of a surgical instrument inside the body are determined on the initial image acquired during a 3D planning imaging exam. For example the above described system and the method for assistance in a surgical procedure may be employed in surgical renal stone removal percutaneous nephrolithotripsy (PCNL) where an access channel to the optimal renal calyx to reach the stone is planned on a 3D-CT scan and then dilated during the surgery through skin and kidney parenchyma to allow insertion of endoscopic instruments for stone fragmentation and removal. Typically, gaining renal access is technically challenging, because the calyx of choice is to be entered at the correct angle to avoid hemorrhage and the kidney moves due to breathing. Furthermore, the PCNL procedure is often performed with the patient in a prone position, while the diagnostic CT is performed with the patient in a supine position. In such surgery, the above-described system and the method for assistance in a surgical procedure may be used to improve the safety for the patient while the required x-ray exposure may be reduced to a minimum.

It shall be understood that the system of claim 1, the method of claim 12, and the computer program of claim 14, have similar and/or identical preferred embodiments, in particular, as defined in the dependent claims.

It shall be understood that a preferred embodiment of the present invention can also be any combination of the dependent claims or above embodiments with the respective independent claim. These and other aspects of the invention will be apparent from and elucidated with reference to the embodiments described hereinafter.

### BRIEF DESCRIPTION OF THE DRAWINGS

In the following drawings:
Fig. 1 shows a system for assistance in a surgical procedure,
Fig. 2 shows a flowchart diagram representing a method for assistance in a surgical procedure,
Fig. 3 shows an initial image visualized by a visualization unit, and
Fig. 4 shows a spatial registration of an initial image and a pre-surgical cone-beam CT with an overlay of an intervention path.

### DETAILED DESCRIPTION OF EMBODIMENTS

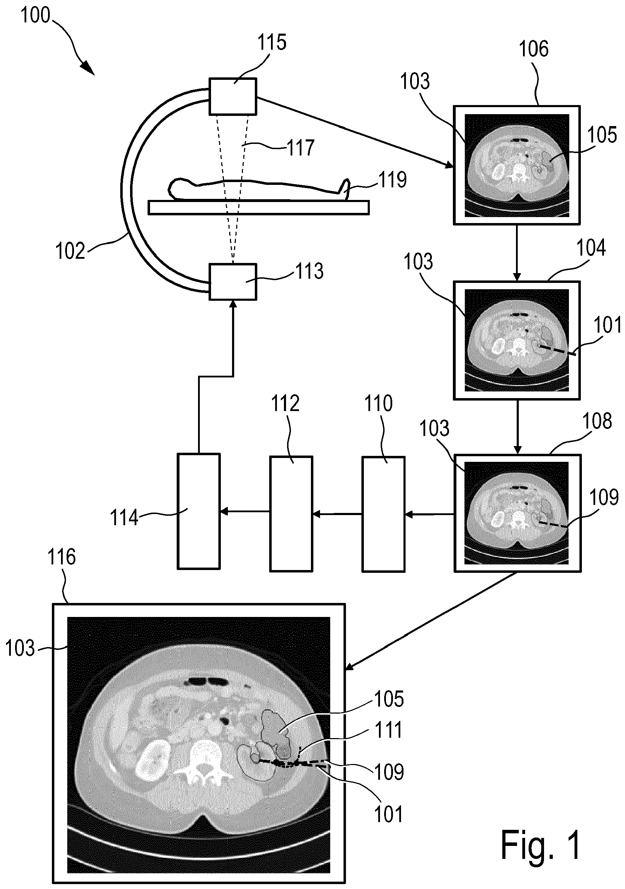

Fig. 1 shows a system 100 for assistance in a surgical procedure. In particular, the system 100 may support a clinician to safely guide a medical instrument (not part of system 100) such as a needle towards a target location, for example, the renal calyx of a kidney.

The system 100 comprises an image providing unit 102 for providing an initial image of a target location inside a patient's body. The image providing unit 102 may be, for example, a fixed or mobile C-arm having an x-ray source 113 and an x-ray detector 115 for detecting x-rays 117 having passed a patient's body 119 (as shown exemplarily in the present case), or a CBCT device or a sonographic device. The C-arm may also be part of the CBCT device. It may also be that the image providing unit 102 is configured for receiving image data representing the initial image from an external imaging device. Accordingly, an imaging device may be the image providing unit 102 or an external imaging device for recording the initial image and may be configured to record projective x-ray images in single exposure or in a fluoroscopy mode, for example, in a continuous fluoroscopy mode. In particular, the initial image is a planning image, for example, a planning CT scan, showing the target location inside a patient's body.

The system 100 further comprises an intervention path providing unit 104 that is configured for processing the initial image 103 provided by the image providing unit 102. In particular, the intervention path providing unit 104 is configured for providing in the initial image 103 and intervention path 101 along which a medical instrument shall be guided to the target location shown in the initial image 103. For example, the intervention path providing unit 104 may comprise software configured for defining the intervention path 101 for the medical instrument. After processing of the initial image 103 with the intervention path providing unit 104, the initial image 103 may be combined with an overlay of the intervention path 101 for insertion of the medical instrument.

The system 100 further comprises a structure at risk providing unit 106 that is configured for processing the initial image 103 to provide in the initial image 103 a location of a structure at risk 105. A structure at risk 105 may be an organ or a vessel that should not be punctured by the medical instrument on its way to the target location. For providing in the initial image 103 a location of a structure at risk 105, the structure at risk providing unit 106 may comprise a neural network that is trained for identifying the location of a structure at risk when provided with the initial image and to output the initial image comprising a label representing the location of a structure at risk in the initial image. After being processed by the intervention path providing unit 104 and by the structure at risk providing unit 106, to the processed initial image 103, an overlay showing the intervention path as well as the location of at least one structure at risk 105 may be added.

Furthermore, the system 100 comprises a position providing unit 108 that is configured for providing a tracked position 109 of the medical instrument. In particular, the position providing unit 108 may receive a tracked position 109 of the medical instrument during a surgical procedure. Accordingly, a received tracked position 109 of the medical instrument represents the position of the medical instrument relative to the patient while conducting the surgery. A graphical representation of the tracked position 109 as provided by the position providing unit 108 may be included in the initial image 103 and updated when receiving a new tracked position 109 with the position providing unit 108. This way, it is possible to follow the tracked position 109 of the medical instrument in the initial image 103 while conducting a surgical procedure. The position providing unit 108 may receive the tracked position 109 from a tracking system (not part of system 100) that is configured to track the position of the medical instrument relative to the patient. In particular, the tracking system tracks the position of the medical instrument, for example, a needle, in relation to the imaging device, such as a C-arm or a CBCT, in the coordinate system of the tracking system. The tracking system may be an optical tracking system, or an electromagnetic tracking system. The tracking system may be configured for tracking the position of the medical instrument in six degrees of freedom. The tracking system may furthermore use knowledge of an x-ray geometry of the imaging system such as a focus-detector distance or a detector active-area size.

The system 100 comprises an evaluation unit 110 that is configured for evaluating whether a special relation between the tracked position 109 as provided by the position providing unit 108 and the location of the structure at risk 105 as provided by the structure at risk providing unit 106 and/or the intervention path 101 as provided by the intervention path providing unit 104 meets a predefined criterion. The predefined criterion used by the evaluation unit 110 for evaluating the special relation may be defined in various ways.

For example, the structure at risk providing unit 106 may define a safety distance with respect to the provided structure at risk in the initial image. The safety distance may be used for generating a margin or envelope closing the structure at risk at least partly at a distance corresponding to the safety distance. The safety distance may be used to define a buffer zone around the structure at risk. One way of defining the predefined criterion may be based on the safety distance in that the predefined criterion is met if the medical instrument is closer to the structure at risk than the defined safety distance. In that case, the medical instrument would be closer to the structure at risk as required by the safety distance such that there would be an increased risk of puncturing the structure at risk. It may be that a defined safety distance actually intersects with the intervention path such that when following the intervention path with the medical instrument, the medical instrument will get closer to the structure at risk then the safety distance. In this case, a threshold intersection distance may be defined with respect to the intersection, e.g., an intersection point, and the predefined criterion may be that the medical instrument is closer to the intersection as provided by the threshold intersection distance. In this case, the medical instrument is actually reaching the intended intersection of the safety distance and the intervention path.

Another way of defining the predefined criterion may be based on a deviation of the tracked position from the intervention path. For example, a threshold deviation value may be defined that is indicative of an allowed deviation of the tracked position from the intervention path. The evaluation unit 110 may now evaluate a deviation of the tracked position with respect to the threshold deviation value and may decide that the special relation meets the predefined criterion if a deviation of the tracked position exceeds the threshold deviation value. In this case, the medical instrument is expected to have moved too far away from the intervention path.

In addition to the just described predefined criteria, a reliability of the tracked position may be determined. For example, the position providing unit 104 may be configured to provide the tracked position together with a reliability value associated with the tracked position. For assessing the reliability, a threshold reliability value may be defined. If the provided reliability value deviates too much from the threshold reliability value, the evaluation unit may provide a warning signal indicative of the reliability value indicating a low reliability. This would mean that the tracking of the position of the medical instrument is performed with an increased uncertainty in relation to the actual position of the medical instrument.

The system 100 further comprises an output unit 112 that is configured for providing an output signal indicative of whether the spatial relation meets the predefined criterion as evaluated by the evaluation unit 110. Optionally the output unit 112 may further provide a warning signal if the right reliability of the tracked position is found to be below the threshold reliability value.

The system 100 may optionally comprise a control unit 114 that may be configured to receive and process the output signal from the output unit 112. In particular, if the output signal indicates that the spatial relation as evaluated by the evaluation unit 110 meets the predefined criterion, the control unit 114 may control the image providing unit 102 to provide a control image of the target location. The control image may be generated by an imaging device that may or may not be the same imaging device that was used for capturing the initial image. In particular, the imaging device used for generating the control image may be the image providing unit 102 itself or an external imaging device such as a C-arm or a CBCT or a sonographic device.

It may be beneficial, if the evaluation unit 110 continuously checks whether the spatial relation meets at least one of the before described predefined criteria. However, in a surgical procedure there may be parts inside a patient's body that are associated with a lower risk of damaging a structure at risk and there may be other parts inside the patient's body that are associated with a higher risk of damaging a structure at risk.

To this end the control unit 114 may be configured for defining one or more control areas that may be control points with a predefined diameter in the initial image that indicate a position of the medical instrument at which the predefined criterion is met. In particular, if the medical instrument reaches a control area, it may be beneficial to provide a control image of the target location. Thereby, a risk of puncturing a structure at risk may be reduced and thus the safety of a surgical procedure improved.

The system 100 may furthermore comprise an optional visualization unit 116 that is configured for visualizing the tracked position 109 of the medical instrument as provided by the position providing unit 106 and one or more control areas as provided by the control unit 114 in the initial image 103. Additionally, the visualization unit 116 may be configured for visualizing the intervention path 101 as provided by the intervention path providing unit 104 and the location of a structure at risk 105 as provided by the structure at risk providing unit 108 in the initial image 103. Furthermore, in addition, the visualization unit 116 may visualize a safety distance or an associated safety margin 111 or safety envelope in the initial image 103.

With system 100, it is thus possible to warn a clinician if the during insertion phase the medical instrument gets dangerously close to a structure at risk such as an organ. This allows replacing continuous x-ray fluoroscopy imaging with a dose-saving regime of intermittent single-view x-ray exposures at a few time points, e.g., control exposures, selected by the system 100. Thereby, the system 100 allows to safely guide the medical instrument to a target location and at the same to reduce the x-ray exposure to a minimum.

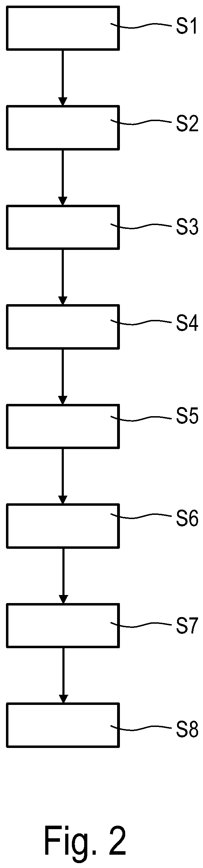

Fig. 2 shows a flowchart diagram representing a method for assistance in a surgical procedure. The method as described in the following may be carried out using the system 100 as described with reference to Fig. 1.

The method starts with a planning phase comprising that an initial image of a target location inside a patient's body is provided (step S1). The initial image can be a planning CT scan and may be used for identifying and delineating all relevant structures. Considering an exemplary case of a kidney stone removal, the initial image may show the kidney as well as anatomical structures such as the urinary collecting system (pyelum and calyxes), lung pleura and ureteral anatomy and the kidney stone. During the planning phase, one or more of such anatomical structures may be identified. In the just mentioned example, the kidney stones would be the target location whereas further anatomical structures may be identified as a structure at risk.

In particular, in the method one or more locations of a structure at risk may be provided (step S2). Structures at risk may be identified in the initial image using a neural network that was trained accordingly. For example, using software, the identified structures at risk may be labelled in the initial image. Preferably, a structure at risk as an organ or an anatomical structure puncturing of which with the medical instrument would be harmful to the patient. Therefore, the intervention path for the medical instrument should not traverse a structure at risk. Additionally to labelling the structures at risk, it may be beneficial to compute a safety margin around an identified structure at risk. The safety margin around a structure at risk may be defined based on the gross spatial extent of expected movement caused by patient positioning, for example, prone patient positioning, and/or expected breathing motion relative to the intervention path. A safety margin may be visualized together with an identified structure at risk as an additional overlay to the initial image. The extent of a safety margin may also be influenced by an expected accuracy of medical instrument tracking and/or registration. Since surgery is typically carried out under general anesthesia, the anesthesiologist may induce apnea to control the motion for when a clinician advances the medical instrument, which may be reflected by a decrease in the extend of a safety margin.

The method further comprises that an intervention path along which a medical instrument shall be guided to the target location is provided (step S3). The intervention path may be visualized together with an identified structure at risk and a safety margin around that structure at risk in the initial image. For example, the intervention path may be input manually and then the intervention path may be provided, for example, by an intervention path providing unit. It is also possible that the intervention path is determined by software, for example, using a trained neural network and then provided by an intervention path providing unit. As part of the planning phase, initially, structures at risk as well as safety margins around the identified structures at risk may be provided in the initial image. For example, the initial image may be visualized together with an overlay of the structures at risk and another overlay with determined safety margins. The intervention path may then be selected and provided such that it has the least contact with the safety margins and the identified structures at risk. Yet, there may be situations in which traversing at least one of a safety margin and a structure at risk may not be avoidable to reach the target location.

Intersections of the intervention path and a safety margin and/or a structure at risk may be computed, for example, using software, and defined as a control area that may be a control point with a predefined diameter. A control area defines a position of the medical instrument at which it is recommended to provide a control image to verify the actual position of the medical instrument in relation to, e.g., a structure at risk.

If a CBCT is used during surgery, at the start of the intervention, a calibration CBCT may be required to determine the transformation T_{CT→CBCT} between the planning CT and CBCT. The accuracy of the registration may depend on various factors such as image quality and field of view of the CBCT, the level of contrast of the target location and the magnitude of the deformation, for example, a nonlinear deformation, between the CT and the CBCT. Considering an example of a kidney intervention, registration can be done by segmenting kidney and inner structures such as calyxes and renal pelvis, and using this information for a coarse rigid alignment of the images. A more precise and, e.g., non-linear alignment can be obtained by a subsequent intensity based refinement of the transformation. Alternatively to performing the planning using a CT, it is possible to use the CBCT at the start of the intervention. In such case, no planning CT as needed and, as a result, no registration component. As a result, providing the structure at risk and the intervention path may be done directly on the CBCT.

During surgery, a position of the medical instrument is tracked, for example, using an optical tracking system, or an electromagnetic tracking system. The tracked position is then provided (step S4), for example, by a position providing unit. The provided tracked position of the medical instrument can be received from the tracking system.

In the method, it is further evaluated whether determined a spatial relation (step S5) between the tracked position and the location of the structure at risk and/or the intervention path meets a predefined criterion (step S6). Furthermore, an output signal is provided that is indicative of whether the spatial relation meets the predefined criterion (step S7). As described before, a predefined criterion may be met if the tracked position of the medical instrument is closer to the structure at risk then a safety distance that is defined relative to the structure at risk and may translate to a safety margin around the structure at risk. Alternatively, the predefined criterion may be if the tracked position is closer to an intersection between the provided intervention path and a defined safety distance then a predefined threshold in the section distance. Again alternatively, the predefined criterion may be met if a deviation of the tracked position from the intervention path exceeds a threshold deviation value indicative of an allowed deviation of the tracked position from the intervention path. Yet, again alternatively, the predefined criterion may be met if the tracked position is closer to the target location then a threshold target distance defined relative to the target location.

In the method, it is further possible that a control image of the target location is provided if the output signal indicates that the evaluated spatial relation meets the predefined criterion (step S8). Accordingly, during the intervention, in the method a recommendation may indicate to provide a control image wherein the recommendation is based on the evaluation of the spatial relation between the tracked position and the structure at risk and/or the intervention path. By means of control areas defined in the initial image there may be a number of planned control exposures for providing the control image, for example, when the medical instrument is near a structure at risk. This may be if the distance of the medical instruments tip to the structure at risk is smaller than, for example, a predefined safety margin. Preferably, another planned control exposure is conducted when the medical instrument is close to the target location, for example, when reaching the kidney prior to removal of a kidney stone.

In addition, in the method it may be further comprised to perform ad-hoc control exposures to provide a control image, for example, if the tracking system or a position providing unit indicates a comparatively high uncertainty about the tracked position. Another reason for performing an ad-hoc control exposure may be if a planned control exposure shows a large deviation from the original surgery plan or an inaccuracy of the registration. For control exposure, it may be beneficial if a C-arm is positioned to allow optimal assessment of the medical instrument position in relation to a structure at risk. For example, a C-arm may be positioned such that the control image is captured in a direction orthogonal to the shortest path between the medical instrument's tip and a surface of the structure at risk. In the method, it may be furthermore preferred, if the intervention path is provided in the initial image in a way such that a minimum number of planned control exposures is required.

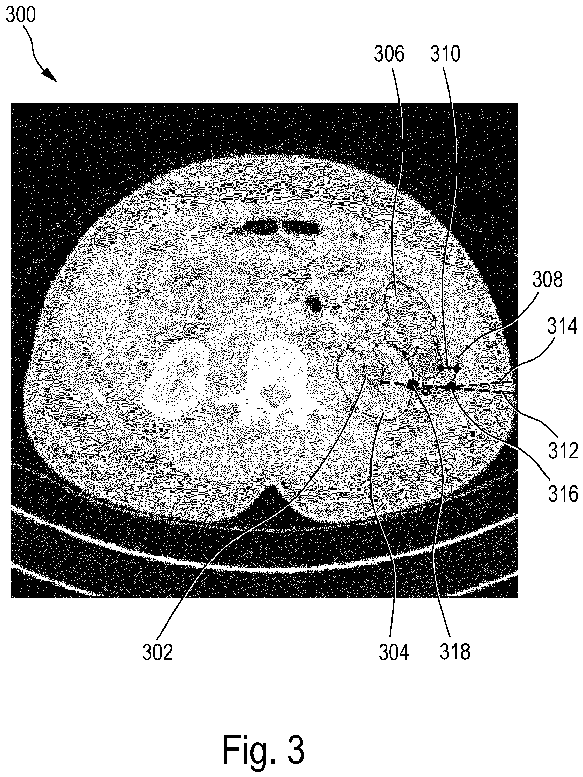

Fig. 3 shows an initial image 300 as visualized by a visualization unit, for example, visualization unit 116 of system 100 is described with reference to Fig. 1. A visualization unit may be or may comprise a graphical user interface (GUI). The initial image 300 is a planning CT scan. As an overlay of the initial image, anatomical structures are identified and labelled. One of these structures is the target location 302 that is a kidney stone of a kidney 304. Another identified anatomical structure is a structure at risk 306 that is partly surrounded by a safety margin 308 that is arranged at a safety distance 310 from the structure at risk 306. Furthermore, the intervention path 312 is provided in the initial image 300. The intervention path 312 represents a route along which the medical instrument shall be guided to reach the target location 302. Accordingly, the intervention path 312 connects an incision point with the target location 302. The actual path of the medical instrument is determined with a tracking system and the tracked position 314 of the medical instrument may be visualized in the initial image 300 during a surgical procedure. This requires a transformation of the coordinate of the medical instrument's position in the coordinate system of the tracking system into the coordinate system of the C-arm that was used for recording the initial image 300. If during surgery, a CBCT is used for recording a control image or a new initial image, the inverse of the transformation T_{CT→CBCT} may need to be determined for visualizing the tracked position of the medical instrument. As can be seen in the initial image 300, the tracked position 314 of the medical instrument does not exactly follow the planned intervention path 312 but slightly deviates from it. If the deviation between the tracked position 314 from the intervention path 312 exceeds a predefined threshold deviation value, an ad-hoc control exposure may be triggered for providing a control image.

Since the intervention path 312 intersects the safety margin 308, a first control area 316 is provided in the initial image 300. The first control area 316 defines a position of the medical instrument at which a planned control image is provided. Hence, if the medical instrument is expected to reach the first control area 316, a control unit may control an image providing unit to provide a control image of the target location 302. A second control area 318 is provided prior to reaching the target location 302 at a position where the medical instrument is expected to puncture the kidney 304. So a planned control image is provided just before reaching the target location 302 to verify the actual position of the medical instrument relative to the target location 302.

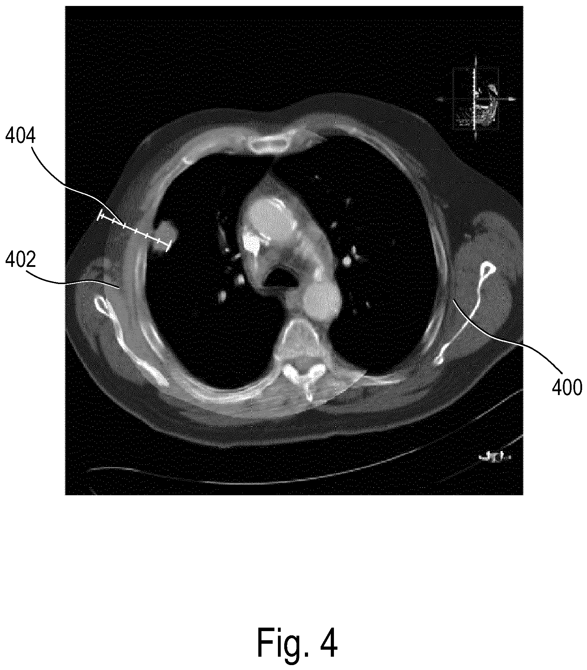

In Fig. 4, a spatial registration of an initial image 400 and a pre-surgical cone-beam CT 402 is shown. Furthermore, an overlay of an intervention path 404 is visualized. To this end, a registration algorithm may be employed to compute the transformation T_{CT→CARM} between CT and C-arm coordinate systems or T_{CT→CBCT} in case a CBCT imaging is used. Optionally, if non-CBCT imaging is used, the application of a tracking system to establish the transformation T_{C-arm→medical} instrument between C-arm and tracking system coordinate systems may be employed.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like providing an initial image, providing in the initial image an intervention path, providing in the initial image a location of a structure at risk, providing a tracked position of the medical instrument, et cetera, performed by one or several units or devices can be performed by any other number of units or devices. These procedures can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a system and to a method for assistance in an image guided surgical procedure, in particular in image guided percutaneous needle interventions such as for kidney stone removal. With the system and the method, it may be possible to provide an automatic recommendation of control scans at critical phases during the intervention. To this end, in an initial image, a structure at risk is identified and an intervention path to a target location is provided. With an evaluation unit, it can be evaluated whether a spatial relation between a) a tracked position of a medical instrument and b) the location of the structure at risk and/or the intervention path meets a predefined criterion. If the predefined criterion is found to be met, an acquisition of a control scan may be recommended automatically.

## Claims

1. A system (100) for assistance in a surgical procedure, the system (100) comprising
- an image providing unit (102) configured for providing an initial image (300) of a target location (302) inside a patient's body,
- an intervention path providing unit (104) configured for providing in the initial image (300) an intervention path (312) along which a medical instrument shall be guided to the target location (302),
- a structure at risk providing unit (106) configured for providing in the initial image (300) a location of a structure at risk (306),
- a position providing unit (108) configured for providing a tracked position (314) of the medical instrument,
- an evaluation unit (110) configured for evaluating whether a spatial relation between a) the tracked position (314) and b) the location of the structure at risk (306) and/or the intervention path (312) meets a predefined criterion, and
- an output unit (112) configured for providing an output signal indicative of whether the spatial relation meets the predefined criterion.

2. The system of claim 1, further comprising a control unit (114) configured for controlling the image providing unit (102) to provide a control image of the target location (302) if the output signal indicates that the evaluated spatial relation meets the predefined criterion.

3. The system of claim 1 or 2, wherein the control unit (114) is configured for defining a control area (316, 318) at which the predefined criterion is met and for controlling the image providing unit (102) to provide a control image when the medical instrument reaches the control area (316, 318).

4. The system of at least one of the preceding claims, further comprising a visualization unit (116) configured for visualizing the tracked position (314) and the control area (316, 318) in the initial image (300), said control area (316, 318) indicating a position of the medical instrument at which the control image has been or will be provided.

5. The system of claim 4, wherein the visualization unit (116) is further configured for visualizing the intervention path (312) and wherein the control area (316, 318) lies on the intervention path (312).

6. The system of at least one of the preceding claims, wherein the structure at risk providing unit (106) is configured for defining a safety distance (310) with respect to the structure at risk (306) and wherein the evaluation unit (110) is configured for evaluating that the spatial relation between a) the tracked position (314) and b) the location of the structure at risk (306) meets the predefined criterion if the tracked position (314) of the medical instrument is closer to the structure at risk (306) than the defined safety distance (310).

7. The system of at least one of the preceding claims, wherein the structure at risk providing unit (106) is configured for defining a safety distance (310) with respect to the structure at risk (306) and for determining an intersection (316) between the provided intervention path (312) and the defined safety distance (310) and wherein the evaluation unit (110) is configured for evaluating that the spatial relation between a) the tracked position (314) and b) the location of the structure at risk (306) and the intervention path (312) meets the predefined criterion if the tracked position (314) is closer to the intersection (316) than a predefined threshold intersection distance.

8. The system of at least one of the preceding claims, wherein the evaluation unit (110) is configured for evaluating that the spatial relation between a) the tracked position (314) and b) the intervention path (312) meets the predefined criterion if a deviation of the tracked position (314) from the intervention path (312) exceeds a threshold deviation value indicative of an allowed deviation of the tracked position (314) from the intervention path (312).

9. The system of at least one of the preceding claims, wherein the evaluation unit (110) is configured for evaluating if a reliability value associated with the tracked position (314) is below a threshold reliability value for the tracked position (314) and the output unit (112) is further configured for providing a warning signal if the reliability value is below the threshold reliability value.

10. The system of at least one of the preceding claims, wherein the evaluation unit (110) is configured for evaluating that the spatial relation between a) the tracked position (314) and the intervention path (312) meets the predefined criterion if the tracked position (314) is closer to the target location (302) than a threshold target distance to the target location (302).

11. The system of at least one of the preceding claims, wherein the structure at risk providing unit (106) comprises a neural network that is trained for receiving the initial image (300) as input and for providing as output a location of the structure at risk (306) in the initial image (300).

12. A method for assistance in a surgical procedure, said method comprising the steps of:
- providing an initial image of a target location inside a patient's body (S1),
- providing in the initial image an intervention path for guiding a medical instrument to the target location (S3),
- providing in the initial image a location of a structure at risk (S2),
- providing a tracked position of the medical instrument (S4),
- determining a spatial relation between the tracked position and the planned intervention path with the determination unit (S5),
- evaluating whether a spatial relation between a) the tracked position and b) the location of the structure at risk and/or the intervention path meets a predefined criterion (S6), and
- providing an output signal indicative of whether the spatial relation meets the predefined criterion (S7).

13. The method of claim 12, further comprising
- providing a control image of the target location if the output signal indicates that the evaluated spatial relation meets the predefined criterion (S8).

14. A computer program including instructions for executing the steps of the method of claim 12 or 13, when run on a computer.

15. A non-transitory computer readable data medium storing the computer program of claim 14.
